# EUROPÄISCHE PATENTSCHRIFT

(11) **EP 0 643 705 B1**
(45) Veröffentlichungstag und Bekanntmachung des Hinweises auf die Patenterteilung: **31.03.1999**
(21) Anmeldenummer: 93912835.1
(22) Anmeldetag: 28.05.1993
(51) Int. Cl.: C07D 257/02, C07D 405/04

(54) **VERFAHREN ZUR HERSTELLUNG VON N-beta-g(b)-HYDROXYALKYL-TRI-N-CARBOXYALKYL-1,4,7,10-TETRAAZACYCLODODECAN- UND N-beta-g(b)-HYDROXYALKYL-TRI-N-CARBOXYALKYL-1,4,8,11-TETRAAZACYCLOTETRADECAN-DERIVATEN UND DEREN METALLKOMPLEXE**
PROCESS FOR PRODUCING N-beta-g(b)-HYDROXYALKYL-TRI-N-CARBOXYALKYL-1,4,7,10-TETRAAZACYCLODODECANE AND N-beta-g(b)-HYDROXYALKYL-TRI-N-CARBOXYALKYL-1,4,8,11-TETRAAZACYCLOTETRADECANE DERIVATIVES AND THEIR METAL COMPLEXES
PROCEDE DE PRODUCTION DE DERIVES DE N-beta-g(b)-HYDROXYALKYLE-TRI-N-CARBOXYALKYLE-1,4,7,10-TETRAAZACYCLODODECANE ET DE N-beta-g(b)-HYDROXYALKYLE-TRI-N-CARBOXYALKYLE-1,4,8,11-TETRAAZACYCLOTETRADECANE ET DE LEURS COMPLEXES METALLIQUES

(30) Priorität: 04.06.1992 DE 4218744
(43) Veröffentlichungstag der Anmeldung: 22.03.1995
(73) Patentinhaber: SCHERING AKTIENGESELLSCHAFT, 13353 Berlin (DE)
(72) Erfinder: TILSTAM, Ulf, D-1000 Berlin 65 (DE); BÖRNER, Helmut, D-1000 Berlin 27 (DE); NICKISCH, Klaus, D-1000 Berlin 49 (DE); GRIES, Heinz, D-1000 Berlin 31 (DE); PLATZEK, Johannes, D-1000 Berlin 33 (DE)
(86) Internationale Anmeldenummer: EP9301362
(87) Internationale Veröffentlichungsnummer: WO9324469

(56) Entgegenhaltungen:
- EP-A- 0 255 471
- EP-A- 0 296 522
- EP-A- 0 382 583
- EP-A- 0 389 359
- EP-A- 0 434 345
- EP-A- 0 434 346
- EP-A- 0 448 191
- EP-A- 0 485 045

## Beschreibung

Die Erfindung betrifft das in den Patentansprüchen gekennzeichnete Verfahren zur Herstellung von N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacydotetradecan-Derivaten und deren Metallkomplexe.

Wegen ihrer Bedeutung zur Herstellung von bildgebenden Diagnostika (DE OS 36 25 417), insbesondere NMR-Diagnostika, ist die Herstellung von N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivaten auf unterschiedlichste Art und Weise versucht worden, ohne daß bisher ein befriedigender Syntheseweg, insbesondere zu ihrer Herstellung im Betriebsmaßstab, gefunden werden konnte.

Für die Synthese von Tetraazamakrocyclen (1,4,7,10-Tetraazacyclododecan-Derivaten oder 1,4,8,11-Tetraazacyclotetradecan-Derivaten) mit dem oben erwähnten Substitutionsmuster werden im Stand der Technik im wesentlichen drei verschiedene Wege beschritten:
1) Man geht von zwei Reaktanden aus, die nach literaturbekannten Methoden [ z.B. Richman, Org. Synthesis 58 86 (1978); Atkins, J. Amer. Chem. Soc. 96 2268 (1974)] zum Tetraazamakrocyclus cyclisiert werden: einer der beiden Reaktanden enthält ein geschütztes Stickstoffatom und trägt am Kettenende zwei Fluchtgruppen ( z.B. Brom-, Mesyloxy-, Tosyloxy-, Triflat- oder Alkoxycarbonylgruppen ), die von den endständigen Stickstoffatomen des zweiten Reaktanden, einer geschützten Triazaverbindung, deren Schutzgruppen sich von der des ersten Reaktanden unterscheiden, nukleophil verdrängt werden. Man erhält so einen tetrasubstituierten Tetraazamakrocyclus mit drei gleichen und einer davon verschiedenen Schutzgruppe. Die Schutzgruppen können nun gezielt abgespalten und die gewünschten Substituenten eingeführt werden. Als Beispiel sei die Umsetzung des Dinatriumsalzes des N,N',N"-Tris (p-tolylsulfonyl)diethylentriamins [Ciampolini, J. Chem. Soc. Chem. Commun. 998 (1984) mit N-Bis-(2-Methansulfonyloxyethyl)triphenylmethylamin in Dimethylformamid bei 80° - 150°C mit nachfolgender Abspaltung der Tritylgruppe unter sauren Bedingungen genannt. Die Ausbeuten der beiden Reaktionsstufen sind im allgemeinen schlecht. Nach der sich anschließenden gezielten Monosubstitution zur Einführung des Substituenten R² [Ciampolini, J. Chem. Soc. Chem. Commun. 998 (1984); Kaden, Helv. Chim. Acta 66 861 (1983); Basefield, Inorg. Chem. 25, 4663 (1986)] werden die Schutzgruppen an drei Stickstoffatomen entfernt z.B. mittels Alkalimetall in Ammoniak [ Helv. Chim. Acta, 56 2216 (1973); Helv. Chim. Acta 59 1566 (1976); J. Org. Chem. 53 3521 (1988)] Lithiumaluminiumhydrid [F. Vögle, Liebigs Ann. Chem. 1344 ( 1977)], Red.-Al® [E.H. Gold, J. Org. Chem. 37 2208, (1972) ] Na-Hg [ M. Kellog, J. Org. Chem. 49 110 (1984) ] Elektrolyse [ M. Hesse, Helv. Chim. Acta 71 (1988), 7, 1708 ] oder Bromwasserstoffsäure/Phenol/Eisessig [ N.G. Lukyanenko, Synthesis, 1988, 355]. Anschließende Trialkylierung mit Halogenessigsäure-Derivaten führt schließlich zum tetrasubstituierten Tetraazamakrocyclus. Die oben aufgeführten Verfahren der Schutzgruppenabspaltung sind im allgemeinen mit schlechten Ausbeuten verbunden, limitieren die Ansatzgröße hinsichtlich der einzusetzenden Reagenzmenge ( z.B. bei der Natrium-Amalgam-Methode) und sind vor allem bei Substituenten, die empfindliche Gruppen ( z.B. Hydroxyalkyl) tragen, nicht anwendbar.
2) Durch statistische Trisubstitution des ungeschützten Tetraazamakrocyclus erhält man in einem anderen Verfahren einen mit drei identischen Resten (z.B. Tosyl-, Benzoyl-, Carboxyethylrest) substituierten Tetraazamakrocyclus, wobei auch mono- und disubstituierte-Produkte entstehen, die durch selektive Fällung, Chromatographie und Kristallisation abgetrennt werden müssen (EP 232 751, EP 292 689). In beiden europäischen Patentanmeldungen wird in der Stufe der statistischen Trisubstitution eine Ausbeute von ca. 23% erhalten. Das bedeutet, daß 77% des sehr teuren Ausgangsmaterials 1,4,7,10-Tetraazacyclododecan verloren gehen. Die darauf folgenden Stufen lassen sich dann wie unter 1) bereits beschrieben anschließen. Alle dem Fachmann bekannten Nachteile einer statistischen Umsetzung wie die oben angeführten geringen Ausbeuten und Trennprobleme ( vor allem bei der Herstellung größerer Substanzmengen) lassen dieses Verfahren nicht vorteilhaft erscheinen.
3) Tweedle und Mitarbeiter beschreiben in der Europäischen Patentanmeldung 292 689, daß ausgehend von der unsubstituierten makrocyclischen Verbindung 1,4,7,10-Tetraazacyclododecan über eine tricyclische Zwischenstufe die N-Formylverbindung erhalten werden kann. Diese noch drei ungeschütze Stickstoffatome tragende Verbindung kann nun mit Halogenessigester-Derivaten trialkyliert, deformyliert und in den tetrasubstituierten Tetraazamakrocyclus überführt werden. Die Anzahl der Reaktionsschritte bis zum Triscarboxymethyl-monoalkyl-tetraazamakrocyclus ist jedoch auch in diesem Verfahren unbefriedigend hoch. Des weiteren hat sich gezeigt, daß die tricyclische Zwischenstufe extrem empfindlich gegen Wasser, Alkohol und Dimethylformamid ist. Diese Stoffe lassen sich bei Großansätzen nicht vollständig genug entfernen, was zu Ausbeuteverlusten führt, die die Anwendbarkeit des Verfahrens im Betriebsmaßstab in Frage stellen.

Es ist also bisher nicht gelungen, einen befriedigenden Syntheseweg für die gewünschten tetrasubstituierten Tetraazamakrocylcen, die als Schlüsselverbindungen für die z. B. als wertvolle NMR- und Röntgen-Kontrastmittel dienenden Tri-N-carboxyalkyl-Metallkomplexe anzusehen sind, zu finden.

Wegen des hohen Bedarfs an NMR- und Röntgenkontrastmitteln und der genannten Nachteile des Standes der Technik besteht daher weiterhin die Aufgabe, ein Verfahren zur Herstellung dieser Mittel, das vor allem für die Umsetzung größerer Substanzmengen geeignet ist, zur Verfügung zu stellen.

Diese Aufgabe wird durch das vorliegende Verfahren gelöst.

Es wurde gefunden, daß man überraschenderweise N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivate der allgemeinen Formel I 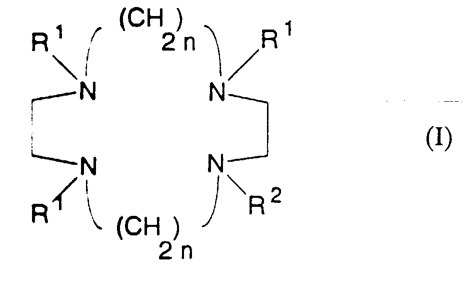 worin
- R¹: eine gegebenenfalls durch R³ substituierte -(CH₂)₁₋₆-COOY-Gruppe mit
R³ in der Bedeutung eines Wasserstoffatoms, einer C₁-C₆ Alkylgruppe, einer Benzyl-, einer Benzyloxyalkyl- oder einer Phenylgruppe,
Y in der Bedeutung eines Wasserstoffatoms und/oder eines Metallionenäquivalentes eines Elements der Ordnungszahlen 21-29, 31, 32, 37-39, 42-44, 49 oder 57-83 eine
- R²: eine 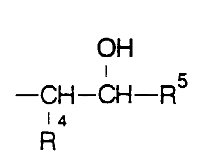 Gruppe
- n: die Ziffern 2 oder 3
- R⁴ und R⁵: unabhängig voneinander jeweils für ein Wasserstoffatom, einen gegebenenfalls durch 1 bis 10 Sauerstoffatom(e), eine Phenylen-, Phenylenoxyoder Phenylendioxygruppe unterbrochenen C₁-C₂₀ Alkylrest, der gegebenenfalls durch 1 bis 3 C₁-C₆-Alkyl, 1 bis 3 Trifluormethyl-, 1 bis 7 Hydroxy-, 1 bis 3 C₁-C₇-Alkoxy- oder -(C₆-C₁₀)Ar-(C₁-C₆)-alkoxy-, 1 bis 2 CO₂R⁶-Reste,
wobei
R⁶ für ein Wasserstoffatom, eine C₁-C₆-Alkylgruppe, eine C₆-C₁₀-Aryl-oder eine C₆-C₁₀-Ar(C₁-C₄)-alkylgruppe steht, und/oder 1 bis 2 gegebenenfalls durch 1 bis 2 Chlor-, Brom-, Nitro- oder C₁-C₆-Alkoxyreste substituierte Phenoxy- oder Phenylgruppen substituiert ist,
bedeuten, wobei die gegebenenfalls vorhandenen Hydroxyreste oder Carboxygruppen gegebenenfalls in geschützter Form vorliegen,
erhält,
wenn man 1,4,7,10-Tetraazacyclododecan oder 1,4,8,11-Tetraazacyclotetradecan, gegebenenfalls in Form ihrer Salze, in Gegenwart einer Base mit einem Epoxid der Formel II, 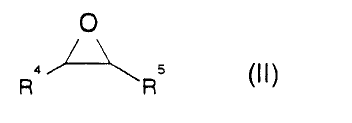 worin
R⁴ und R⁵ die oben angegebenen Bedeutungen haben, wobei gegebenenfalls vorhandene Hydroxy- oder Carboxygruppen gegebenenfalls geschützt sind,
in einem polaren Lösungsmittel oder ohne Lösungsmittel bei Temperaturen von 0° - 220°C innerhalb von 0.5 - 48 Stunden umsetzt, die Verunreinigungen abtrennt, gewünschtenfalls, wenn notwendig nach Zugabe von Säuren, isoliert, und gegebenenfalls in Gegenwart einer Base, mit einer Verbindung der Formel III 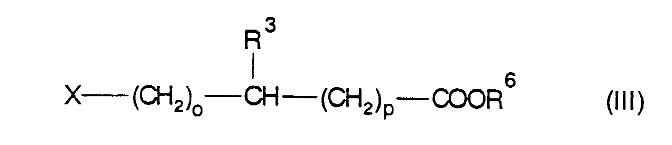 worin
- R³ und R⁶: die oben angegebene Bedeutung haben,
- X: eine Fluchtgruppe, und
- o,p: unabhängig voneinander die Ziffern 0 bis 5, wobei die Summe aus o+p<6 sein muß, bedeuten,
gegebenenfalls nach Schutz von Hydroxy- oder Carboxygruppen in R², in einem polaren Lösungsmittel bei -10°C bis 170°C innerhalb von 1 - 100 Stunden umsetzt, gewünschtenfalls Schutzgruppen abspaltet und das so erhaltene Produkt der Formel I in dem Y Wasserstoff bedeutet, gewünschtenfalls in an sich bekannter Weise mit mindestens einem Metalloxid oder Metallsalz eines Elements der Ordnungszahlen 21-29, 31, 32, 37-39, 42-44, 49 oder 57-83 umsetzt, falls gewünscht, noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt und den so erhaltenen Komplex isoliert.

Das erfindungsgemäße Verfahren zeichnet sich im Hinblick auf den Stand der Technik durch mehrere entscheidende Vorteile aus.
1) Die Verwendung von Stickstoffschutzgruppen wird gänzlich vermieden.
2) Die Tetraazamakrocyclen, die empfindliche Gruppen wie z.B. Hydroxygruppen, tragen, werden durch das vorliegende Verfahren auch im Großmaßstab herstellbar.
3) Die Extraktionsschritte im Anschluß an die Umsetzung mit dem Epoxid der Formel II ermöglichen eine vollständige Abtrennung der Nebenprodukte, so daß aufwendige chromatographische Trennungen oder selektive Fällungen entfallen.
4) Das erfindungsgemäße Verfahren führt in erheblich geringerer Stufenzahl zum tetra substituierten Makrocyclus als die Verfahren des Standes der Technik.
5) In die erste Reaktionsstufe wird maximal ein Äquivalent Makrocyclus im Verhältnis zum Epoxid eingesetzt und das Substitutionsprodukt in hohen Ausbeuten erhalten. So mit wird ein großer Verlust des sehr teuren Ausgangsmaterials 1,4,7,10-Tetraazacyclo dodecan vermieden.

Die in EP-A-485045 und EP-A-448191 offenbarten Verfahren zur Synthese von tetrasubstituierten 1,4,7,10-Tetraazacyclododecan-Derivaten verlaufen unter Verwendung von Schutzgruppen über eine Alkylierung einer trisubstituierten Ausgangsverbindung. Das erfindungsgemäße Verfahren der Monoalkylierung eines unsubstituierten Tetraazamakrocyclus' und anschließender Einführung der drei Essigsäuregruppen R¹ weist gegenüber diesen Verfahren die unter 1 bis 4 aufgeführten Vorteile auf.

Die Carboxyalkylgruppe R¹ kann unverzweigt oder verzweigt sein, wobei unverzweigte Carboxyalkylgruppen bevorzugt werden. Die Länge der Alkylkette kann 1 bis 6 Kohlenstoffatome, bevorzugt 1 bis 2 Kohlenstoffatome, betragen.

Als Alkylgruppe R⁶ und R³ mit 1-6 Kohlenstoffatomen kommen geradkettige oder verzweigte Alkylgruppen in Betracht wie beispielsweise Methyl, Ethyl, Propyl, Isopropyl, Butyl, Isobutyl, tert.-Butyl. Besonders bevorzugt werden Methyl, Ethyl, tert.-Butyl.

Bevorzugte Reste R⁴ und R⁵ sind das Wasserstoffatom, die Methyl-, Ethyl-, 2-Hydroxyethyl-, 2-Hydroxy-1-(hydroxymethyl)ethyl-, 1-(Hydroxymethyl)ethyl-, Propyl-, Isopropyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-Hydroxy-2-methylbutyl-, 3-Hydroxy-2-methylbutyl-, 4-Hydroxy-2-methylbutyl-, 2-Hydroxyisobutyl-, 3-Hydroxyisobutyl-, 2,3,4-Trihydroxybutyl-, 1,2,4-Trihydroxybutyl-, Pentyl-, Cyclopentyl-, 2-Methoxyethyl-, Hexyl-, Decyl-, Tetradecyl-, Triethylenglycolmethylether, Tetraethylenglykolmethylether- und Methoxybenzylgruppe sowie die
-CH₂-O-C₁₁H₂₂-OH-,
-CH₂-O-C₆H₄-O-(CH₂CH₂O)₂-CH₃-
-CH₂-O-C₆H₄-O-(CH₂CH₂O)₃-C₅H₁₁-,
-CH₂-O-C₆H₄-O-C₄H₈-OH-,
-(CH₂CH₂O)₅-CH₃-,
-C₉H₁₈-COOH-,
-C₉H₁₈-OH-,
-CH₂-O-C₆H₄-O-C₆H₁₂-COOH-,
-CH₂-O-C₆H₄-O-C₄H₈-O-CH₂-CHOH-CH₂OH-,
-(CH₂CH₂-O)₃-C₅H₁₁-,
-CH₂-O-C₁₀H₂₀-COOH-, -CH₂-O-C₆H₄-Cl-,
-CH₂-O-C₆H₄-NO₂-,
-CH₂-O-C₆H₃Cl₂-,
-CH₂-O-C₆H₄-O-CH₂-COOH-,
-CH₂-O-C₆H₄-O-CH₂-COOH- und
-CH₂-O-C₆H₄-C₅H₁₁-Gruppe.

Bevorzugte Arylgruppen und Aralkylgruppen R⁶ sind die Phenyl-, Naphthyl- und die Benzylgruppe.

Besonders bevorzugte Reste R⁶ sind Wasserstoff, der Methylrest oder der Benzylrest.

Besonders bevorzugte Reste R³ sind Wasserstoff, der C₁-C₃-Alkylrest oder der Benzyloxymethylrest.

Im Epoxid II gegebenenfalls vorhandene Carboxyl- und/oder Hydroxygruppen liegen vorzugsweise in geschützter Form vor.

Als Säureschutzgruppen, die auch für R⁶ stehen, kommen niedere C₁-C₆-Alkyl-, C₆-C₁₀-Aryl- und C₆-C₁₀-Ar(C₁-C₄)-alkylgruppen, beipielsweise die Methyl-, Ethyl-, Propyl-, n-Butyl, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen erfolgt nach den dem Fachmann bekannten Verfahren, beipielsweise durch Hydrolyse, Hydrogenolyse, alkalische Verseifung der Ester mit Alkali in wäßrig-alkoholischer Lösung bei Temperaturen von 0 bis 50°C, saure Verseifung mit Mineralsäuren oder im Fall von z.B. tert.-Butylestern mit Hilfe von Trifluoressigsäure.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl-, Diphenyl-t-butylsilyl-gruppen infrage.

Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle von Polyolen können die Hydroxygruppen auch in Form von Ketalen mit z.B. Aceton, Acetaldehyd, Cyclohexanon oder Benzaldehyd geschützt sein.

Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthetics", T.W. Greene, John Wiley and Sons 1981).

Für die Fluchtgruppe X kann jede dem Fachmann geläufige Abgangsgruppe stehen. Beispielsweise seien Acetat, Brosylat, Mesylat, Nosylat, Tosylat, Trifluoracetat, Trifluorsulfonat, Chlor, Brom oder Jod genannt. Bevorzugte Fluchtgruppen sind Chlor und Brom, besonders bevorzugt ist Chlor.

Als Ausgangsverbindung werden für das erfindungsgemäße Verfahren die makrocyclischen Verbindungen 1,4,7,10-Tetraazacyclododecan oder 1,4,8,11-Tetraazacyclotetradecan oder deren Salze eingesetzt.

Es kommen als Salzbildner alle anorganischen und organischen Säuren in Frage, die mit den genannten Makrocyclen stabile Salze bilden. Beispielsweise seien Phosphorsäure, Salzsäure, Schwefelsäure oder p-Toluolsulfonsäure genannt.

In einem bevorzugten Verfahren werden die genannten Makrocyclen als Hydrochloride oder als Sulfate eingesetzt. Diese können nach literaturbekannten Verfahren erhalten werden. Das Sulfat kann z. B. nach Organic Synthesis, Vol. 58, 89, 1978 erhalten werden. Es wird jedoch in der Literatur als "Polyhydrosulfat" bezeichnet, deren Gehalt durch Schwefelbestimmung nach jedem Ansatz festgestellt werden muß. Der Gehalt schwankt zwischen 3 und 4 Äquivalenten Schwefelsäure.
Das Tetrahydrochlorid kann nach J. Amer. Chem. Soc., 96 2268, (1974) oder nach Recueil des Traveaux de Pays Bas, 110 124, (1991) erhalten werden.

Das molare Verhältnis von Makrocyclus zu Epoxid beträgt nach dem erfindungsgemäßen Verfahren 1:1 bis 1:2, wobei ein Überschuß an Epoxid bevorzugt wird.

Die bei der Umsetzung des Makrocyclus mit dem Epoxid der Formel II 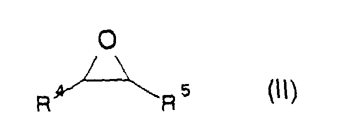 zugesetzte Base kann eine der üblichen dem Fachmann bekannten anorganischen oder organischen Basen sein wie zum Beispiel Kaliumhydroxid, Natriumhydroxid, Lithiumhydroxid, Bariumhydroxid, Calciumhydroxid, Pyridin oder N,N-Dimethylaminopyridin. Bevorzugt werden Kaliumhydroxid, Natriumhydroxid, Lithiumhydroxid, besonders bevorzugt wird Natriumhydroxid verwendet.

Soll die Reaktion ohne Lösungsmittel durchgeführt werden, muß die freie makrocyclische Verbindung umgesetzt werden. Diese kann z.B. analog zu Helv. Chim. Acta, 66 863 (1983) aus dem Sulfat durch Basenbehandlung erhalten werden.

Als Lösungsmittel sind polare aprotische Lösungsmittel wie z.B. Acetonitril, Diethylcarbonat, Diethylether, Dimethylacetamid, Dimethylsulfoxid, Dioxan, N-Methylpyrrolidon, Tetrahydrofuran oder Tetramethylharnstoff und deren Gemische sowie protische Lösungsmittel wie zum Beispiel Alkohole mit 1-8 C-Atomen geeignet. Es seien Methanol, Ethanol, Propanol, Isopropanol, n-Butanol, Isobutanol, tert.-Butanol beispielsweise genannt.

Wenn die für diese Umsetzung verwendeten Lösungsmittel mit Wasser mischbar sind, so werden sie nach Reaktionsende vor einer Extraktion destillativ entfernt.

Bei ausreichender Löslichkeit der Reaktanden können auch relativ unpolare aprotische Lösungsmittel wie zum Beispiel Benzol, Toluol oder Kohlenwasserstoffe wie zum Beispiel n-Hexan für die Umsetzungen verwendet werden.

Die Umsetzung mit dem Epoxid der Formel II wird bei Temperaturen zwischen 0°-220°C. bevorzugt zwischen Raumtemperatur und 200°C, besonders bevorzugt zwischen 50°C und 150°C durchgeführt. Die Reaktionszeit im jeweils entsprechenden Temperaturintervall beträgt 5 bis 48-, bevorzugt 1 bis 24-, besonders bevorzugt 1 bis 6 Stunden.
Die nach Reaktionsende durchgeführte Reinigung des Monoalkylierungsproduktes kann z.B. durch Extraktion erfolgen, gegebenenfalls in mehreren Stufen.

Es ist ein Vorteil des erfindungsgemäßen Verfahrens, daß die Isolierung des Monoalkylierungsproduktes nicht notwendig ist.

Wird jedoch die Spaltung von Schutzgruppen und/oder eine Isolierung des Monoalkylierungsproduktes gewünscht wird, ist es vorteilhaft, Mineralsäuren oder organische Säuren wie zum Beispiel Salzsäure, Schwefelsäure, Ameisensäure, Essigsäure oder Trifluoressigsäure zuzusetzen.

Die nachfolgende Reaktion zur Einführung der drei Carboxyalkylgruppen erfolgt durch Umsetzung mit einer Verbindung der allgemeinen Formel III 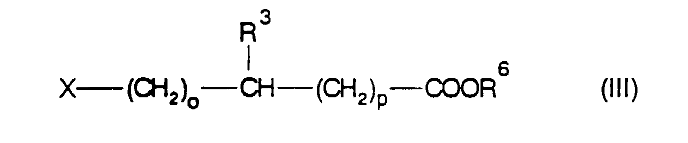 in polaren Lösungsmitteln wie zum Beispiel Acetonitril, Aceton, Dimethylformamid, Dimethylsulfoxid, Hexamethylphosphorsäuretriamid, Tetrahydrofuran oder Wasser oder in Alkoholen mit einer Kettenlänge mit bis zu 8 C-Atomen wie sie bereits für die erste Umsetzung beschrieben worden sind. Bevorzugt werden Dimethylformamid und Wasser.

Die Umsetzung wird bei Temperaturen von -10°C- 170°C, bevorzugt bei 0° - 120°C. besonders bevorzugt bei 40° - 100°C durchgeführt.

Die Reaktionsdauer beträgt zwischen 1 und 100 Stunden, bevorzugt zwischen 1 und 30 Stunden, besonders bevorzugt zwischen 3 und 12 Stunden.

In einem besonders bevorzugten Verfahren ist die Verbindung der Formel III Chloressigsäure.

Die bei der Umsetzung mit der Verbindung der Formel III als Säurefänger zugesetzten Basen können tertiäre Amine (zum Beispiel Triethylamin, Trimethylamin, N,N-Dimethylaminopyridin, 1,5-Diazabicyclo[4.3.0]-nonen-5(DBN), 1,5-Diazabicyclo[5.4.0]-undecen5-(DBU), Alkali- Erdalkalicarbonate, -hydrogencarbonate, oder -hydroxide (zum Beispiel Lithium-, Natrium-, Kalium, Magnesium-, Calcium-, Barium-, -carbonat, -hydroxid und - hydrogencarbonat) sein. Besonders bevorzugt wird Natriumhydroxid verwendet.

Die gegebenenfalls notwendige Einführung oder Spaltung von Schutzgrupen der Carboxyl- oder Hydroxyfunktionen erfolgt nach den bereits für den ersten Verfahrensschritt erwähnten Methoden.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 37-39, 42-44, 49 oder 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Einführung der gewünschten Metallionen kann dabei sowohl vor als auch nach der Abspaltung der Schutzgruppen für die gegebenenfalls vorhandenen Hydroxy- oder anderen funktionellen Gruppen erfolgen.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Lithium, Natrium, Kalium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutral punkt erreicht wird. Die erhaltene Lösung kann anschließend im Vakuum zur Trockene eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrenschritt zu sparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Metallion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (zum Beispiel durch erschöpfende Umsetzung der freien Carboxy-Gruppen mit Dimethylsulfat). Werden die verbleibenden Säuregruppen nur teilweise in Ester oder Amide überführt, können die dann noch verbleibenden freien Säuregruppen wie oben beschrieben in ihre Salze überführt werden.

Die folgenden Ausführungsbeispiele dienen zur Erläuterung der vorliegenden Erfindung, sollen diese jedoch nicht einschränken.

Für die Ionenaustauschchromatographie werden verschiedene Ausführungen des Produktes Amberlite® der Firma Rohm & Haas verwendet.

### Beispiel 1

### Beispiel 1a

### 10-(1-Hydroxymethyl-2,3-dihydroxypropyl)-1,4,7,10-tetraazacyclododecan Tetrahydrochlorid

Zu 282.3 g (500 mmol) 1,4,7,10-Tetraazacyclododecantetrasulfat in 1200 ml n-Butanol werden 120 g (3 mol) Natriumhydroxid unter Stickstoffatmosphäre gegeben. Das Gemisch wird erwärmt und entstehendes Wasser azeotrop abdestilliert. Anschließend werden 86.5 g (600 mmol) 4,4-Dimethyl-3,5,8-trioxabicyclo[5,1,0]-octan in 200 ml Butanol zugetropft. Die Reaktionslösung wird zwei Stunden am Rückfluß erhitzt und nochmals mit 21.6 g (150 mmol) 4,4-Dimethyl-3,5,8-trioxabicyclo[5,1,0]-octan versetzt. Nach weiteren zwei Stunden Rückfluß wird auf Raumtemperatur abgekühlt. Das Reaktionsgemisch wird mit 1000 ml Wasser versetzt und 30 Minuten gerührt. Die Phasen werden getrennt.Die Butanolphase wird mit 250 ml konzentrierter Salzsäure versetzt, eine Stunde bei 70°C gerührt und auf 200 ml im Vakuum eingeengt. Es werden 1000 ml absolutes Methanol zugesetzt. Nach Eingeengen werden nochmals 500 ml absolutes Methanol zugesetzt. Die Lösung wird in Eis/Wasser abgekühlt, und die ausfallenden werden Kristalle abgesaugt. Die Kristalle werden einmal mit Butanol und zweimal mit Methyl-tert.-butylether ( MTB-ether) gewaschen und getrocknet. Das Produkt verliert beim Trocknen HCI. Man erhält 200.5 g ( 95% d. Th.) 10-(1-Hydroxymethyl-2,3-dihydroxypropyl)-1,4,7,10-tetraazacyclododecan Tetrahydrochlorid als weiße Kristalle.
Schmelzpunkt: 214°-216°C

| | | | | | | | | | |
|---|---|---|---|---|---|---|---|---|---|
| Elementaranalyse | Ber. | C | 34.13 | H | 7.64 | N | 13.27 | Cl | 33.59 |
| | Gef. | C | 35.19 | H | 7.85 | N | 13.67 | Cl | 29.61 |

### Beispiel 1b

### 10-(1-Hydroxymethyl-2,3-dihydroxypropyl)-1,4,7-triscarboxymethyl-1,4,7,10tetraazacyclododecan

105.5 g (250 mmol) der in Beispiel la erhaltenen Verbindung werden in 422 ml Wasser gelöst und mit 118.1 g (1.25 mol) Chloressigsäure versetzt. Mit Kaliumhydroxid wird auf pH = 10 eingestellt. Die Reaktionslösung wird bei 70°C vier Stunden gerührt und der pH-Wert auf 9-10 gehalten. Dann wird nach Zugabe von 23.6 g Chloressigsäure weitere zwölf Stunden bei diesen Bedingungen gerührt. Nach Abkühlen auf Raumtemperatur wird mit konzentrierter Salzsäure bis pH = 0.8 angesäuert. Die Lösung wird zur Trockene eingeengt, der Rückstand mit 400 ml eines Gemisches Methanol/Ethanol 1:1 versetzt und erneut eingeengt. Nach Wiederholung dieses Vorganges wird der Rückstand mit 1000 ml Methanol versetzt, 90 Minuten bei 50°C gerührt und auf 0°C abgekühlt. Das ausfallende Kaliumchlorid wird zweimal mit Methanol gewaschen. Die vereinigten Filtrate werden im Vakuum zur Trockene eingeengt. Es werden 176 g Rohprodukt erhalten. Dieses wird in 200 ml entionisiertem Wasser gelöst und über eine Säule mit 2.71 Amberlite®AMB 252c gegeben. Die Säule wird mit entionisiertem Wasser gewaschen bis im Eluat keine Leitfähigkeit mehr nachgewiesen werden kann. Anschließend wird das Produkt mit Wasser/Ammoniak eluiert. Die Produkt enthaltenden Fraktionen werden vereinigt und zur Trockene eingeengt. Es werden 118 g ( = 105.6 g Produkt ohne Wasser, 93% d. Th.)10-(1-Hydroxymethyl-2,3-dihydroxypropyl)-1,4,7 1,4,7,10-tetraazacyclododecan erhalten.

| | | | | | | | |
|---|---|---|---|---|---|---|---|
| Elementaranalyse (auf Wasser korrigiert) | Ber. | C | 47.98 | H | 7.61 | N | 12.43 |
| | Gef.: | C | 47.35 | H | 7.63 | N | 12.32 |

Wassergehalt: 10.51%

### Beispiel 1c

### Gadolinium-Komplex von 10-(1-Hydroxymethyl-2,3-dihydroxypropyl)-1,4,7-triscarboxymethyl-1,4,7,10-tetraazacyclododecan

118 g der aus Beispiel 1b erhaltenen Verbindung werden in 530 ml entionisierten Wasser gelöst und mit 44.5 g (123 mmol) Gadoliniumoxid versetzt. Die Lösung wird zwei Stunden auf 95°C erwärmt, abgekühlt und mit je 30 ml saurem Ionenaustauscher (Amberlite® IR 120) und 30 ml basischem Ionenaustauscher (Amberlite®IRA 67 ) eine Stunde bei Raumtemperatur gerührt. Anschließend wird die Lösung filtriert und das Filtrat kurz mit Aktivkohle aufgekocht. Nach erneuter Filtration wird eingeengt und aus Ethanol/Wasser umkristallisiert.
Es werden 105 g (74% d. Th.) des Gadolinium-Komplexes von 10(1-Hydroxymethyl-2,3-dihydroxypropyl)-1,4,7-triscarboxymethyl-1,4,7,10-tetraazacydododecan erhalten.
Schmelzpunkt: > 300°C

| | | | | | | | | | |
|---|---|---|---|---|---|---|---|---|---|
| Elementaranalyse | Ber. | C | 35.75 | H | 5.17 | N | 9.27 | Gd | 26.00 |
| | Gef. | C | 35.63 | H | 5.15 | N | 9.25 | Gd | 25.97 |

### Beispiel 2

### Beispiel 2a

### 10-(1-Hydroxymethyl-2,3-dihydroxypropyl)-1,4,7-triscarboxymethyl-1,4,7,10-tetraazacyclododecan

Zu 141.1 g (250 mmol) 1,4,7,10-Tetraazacyclododecantetrasulfat in 600 ml n-Butanol werden 68 g (1.7 mol) Natriumhydroxid gegeben. Das Gemisch wird erwärmt und azeotrop Wasser abdestilliert. Anschließend werden 55,0 g (382 mmol) 4,4-Dimethyl-3,5,8-trioxabicyclo-[5,1,0]-octan zugetropft. Nach beendeter Zugabe wird eine Stunde unter Rückfluß gekocht. Danach wird auf Raumtemperatur abgekühlt, mit 500 ml Wasser versetzt und 30 Minuten gerührt. Die Phasen werden getrennt und die Butanolphase zur Trockene eingeengt. Der Rückstand wird in 600 ml Wasser aufgenommen und dreimal mit Ethylacetat extrahiert. Die Wasserphase wird mit 95 g Chloressigsäure versetzt und auf pH 10 gebracht. Nach Zugabe von 159 g Na₂CO₃ wird auf 80°C erwärmt und vier Stunden gerührt. Anschließend werden 20 g Chloressigsäure zugegeben, und es wird weitere zwölf Stunden bei 80°C gerührt. Das Reaktionsgemisch wird auf Raumtemperatur abgekühlt, mit konzentrierter Salzsäure auf pH 0.8 eingestellt, auf 60°C erwärmt und eine Stunde nachgerührt. Danach wird zur Trockene eingeengt, mit 400 ml eines Gemisches Methanol/Ethanol 1:1 versetzt und erneut eingeegt. Dieser Vorgang wird wiederholt, der Rückstand in 1000 ml Methanol aufgenommen, 90 Minuten bei 50°C gerührt und auf 0°C abgekühlt. Das ausfallende Kaliumchlorid wird abgesaugt und zweimal mit Methanol gewaschen. Die vereinigten Filtrate wird im Vakuum zur Trockene eingeengt. Die Ausbeute an Rohprodukt beträgt 176 g. Es wird nun in 200 ml entionisiertem Wasser gelöst und über eine Säule mit 2.7 1 Amberlite®AMB 252c gegeben. Die Säule wird mit entionisiertem Wasser gewaschen bis keine leitfähigkeit im Eluat mehr nachzuweisen ist. Anschließend wird das Produkt mit Wasser/Ammoniak eluiert. Die substanzhaltigen Fraktionen werden vereinigt und zur Trockene eingeengt. Es werden 105 g (93% d. Th.) 10-(1-Hydroxymethyl-2,3-dihydroxypropyl)-1,4,7-triscarboxymethyl-1,4,7,10-tetraazacyclododecan erhalten.

| | | | | | | | |
|---|---|---|---|---|---|---|---|
| Elementaranalyse (auf Wasser korrigiert) | Ber. | C | 47.98 | H | 7.61 | N | 12.43 |
| | Gef. | C | 47.15 | H | 7.72 | N | 12.39 |

Wassergehalt: 95%

### Beispiel 2b

### Gadolinium-Komplex von 10-(1 Hydroxymethyl-2,3-dihydroxypropyl)-1,4,7-triscarboxymethyl-1,4,7,10-tetraazacyclododecan

105 g der unter Beispiel 2a erhaltenen Verbindung werden in 500 ml entionisiertem Wasser gelöst und mit 40.5 g Gadoliniumoxid versetzt. Anschließend wird wie unter Beispiel 1c beschrieben gearbeitet. Es werden 109 g (72% d. Th.) des Gadolinium-Komplexes von 10-(1-Hydroxymethyl-2,3-dihydroxypropyl)-1,4,7-triscarboxymethyl- 1,4,7,10-tetraazacyclododecan erhalten.
Schmelzpunkt: >300°C

| | | | | | | | | | |
|---|---|---|---|---|---|---|---|---|---|
| Elementaranalyse | Ber. | C | 35.75 | H | 5.17 | N | 9.27 | Gd | 26.00 |
| | Gef. | C | 35.59 | H | 5.11 | N | 9.28 | Gd | 25.98 |

### Beispiel 3

### Beispiel 3a

### 10-(6-Hydroxy-2,2-dimethyl-1,3-dioxepan-5-yl)-1,4,7,10-tetraazacyclododecan

Zu 124.0 g (250 mmol) Tetraazacydododecansulfat in 600 ml n-Butanol werden 56.1 g (1.4 mol) Natriumhydroxid gegeben. Das Gemisch wird erwärmt und entstehendes Wasser azeotrop abdestilliert. Anschließend werden 43.25 g (300mmol) 4,4-Dimethyl-3,5,8-trioxabicyclo[5,1,0]-octan in 100 ml Butanol zugegeben. Das Reaktionsgemisch wird eine Stunde am Rückfluß erhitzt und nochmals mit 8.25 g Natriumhydroxid versetzt. Entstehendes Wasser wird abdestilliert und anschließend nochmals mit 7.2 g 4,4-Dimethyl-3,5,8-trioxabicyclo[5,1,0]-octan versetzt. Nach 30 Minuten am Rückfluß Erhitzen wird auf 50°C abgekühlt und mit 400 ml entionisiertem Wasser versetzt. Nach 15 Minuten bei 40°C wird die erste Wasserphase abgetrennt. Die Butanolphase wird mit 300 ml Wasser und 3.75 ml Eisessig versetzt und 15 Minuten nachgerührt. Dann wird die zweite Wasserphase abgetrennt. Danach wird die Butanolphase zweimal mit je 200 ml Wasser und 3.75 ml Eisessig extrahiert und einmal mit 200 ml Wasser ausgerührt. Die Wasserphasen 2-5 werden vereinigt, mit 12.5 ml 50%iger Natriumhydroxidlösung versetzt und zweimal mit je 150 ml und zweimal mit je 100 ml Butanol ausgeschüttelt. Die vereinigten Waschbutanolphasen werden zweimal mit je 100 ml Wasser gewaschen. Die gesamten Butanolphasen werden vereinigt und mit 300 ml Wasser versetzt, Unter Rühren werden 12.75 ml Eisessig zugetropft. Nach 30 Minuten Rühren wird die Wasserphase abgetrennt. Die Butanolphase wird noch zweimal mit je 200 ml Wasser extrahiert. Die vereinigten drei Wasserphasen werden zweimal mit je 100 ml Methylenchlorid gewaschen. Es wird eine Probe von ca. 100 ml gezogen, deren Gehalt über HPLC gegen externen Standard bestimmt, sowie der Wassergehalt der getrockneten Probe ermittelt. Man erhält 61.3 g ( 72% d. Th. )10-(6-Hydroxy-2,2-dimethyl-1,3-dioxepan-5-yl)- 1,4,7,10-tetraazacyclododecan.
Wassergehalt: 4.8%

### Beispiel 3b

### 10-(1-Hydroxymethyl-2,3-dihydroxypropyl)-1,4,7-triscarboxymethyl-1,4,7,10-tetraazacyclododecan

Zu 60.7 g (192 mmol) des aus Beuspiel 3a erhaltenen Rohproduktes in 500 ml entionisiertem Wasser werden 72.7 g (767 mmol) Chloressigsäure unter Eis/Wasser Kühlung und Stickstoffatmosphäre gegeben. Danach wird der pH-Wert=9.5 mit ca. 42 ml 10 molare Natriumhydroxidlösung eingestellt. Die Reaktionslösung wird auf 70°C erwärmt und dabei der pH-Wert bei 9.1-9.5 durch Zugabe von 10 molare Natriumhydroxidlösung gehalten. Während 4 Stunden Nachrührzeit bei 70°C wird der pH-Wert gehalten. Die Reaktionsmischung wird auf 30°C abgekühlt und mit 97 ml konz. HCI auf pH=0.8 angesäuert, auf 60 °C erhitzt und eine Stunde bei dieser Temperatur nachgerührt. Anschließend wird im Vakuum bei dieser Temperatur zur Trockne eingeengt. Der Rückstand wird zweimal mit je 310 ml MeOH/EtOH versetzt und jeweils wieder zur Trockne eingeengt. Der Rückstand wird dann mit 620 ml Methanol versetzt und bei 50°C 30 Minuten gerührt, in Eis/Wasser gekühlt und abgesaugt, NaCl-Rückstand wird mit eiskaltem Methanol nachgewaschen. Das Filtrat wird im Vakuum zur Trockne eingeengt. Das so erhaltene Rohprodukt wird in 133 ml entionisiertem Wasser gelöst, über eine Säule (2.0 1 Amberlite®AMB 252c) gegeben. Die Säule wird mit entionisiertem Wasser gewaschen bis im Eluat eine Leitfähigkeit von 23 µS nachgewiesen wird. Anschließend wird das Produkt mit Wasser/NH₃ eluiert. Die Produkt enthaltenden Fraktionen werden vereinigt und zur Trockne eingeengt. Es werden 68.2 g (78% d. Th. ) 10-(1-Hydroxymethyl-2,3-dihydroxypropyl)-1,4,7,10-tetraazacyclododecan erhalten.

| | | | | | | | |
|---|---|---|---|---|---|---|---|
| Elementaranalyse | Ber. | C | 47.98 | H | 7.61 | N | 12.43 |
| | Gef. | C | 47.63 | H | 7.93 | N | 12.57 |

### Beispiel 3c

### Gadolinium-Komplex von 10-(1-Hydroxymethyl-2,3-dihydroxypropyl)-1,4,7-triscarboxymethyl-1,4,7,10-tetraazacyclododecan

68.0 g der aus Beispiel 3b erhaltenen Verbindung werden in 306 ml entionisiertem Wasser gelöst, mit 24.65 g (68.1 mmol) Gadoliniumoxid versetzt und auf 95°C erwärmt. Nach 45 Minuten bei dieser Temperatur wird 2.74 g Gadoliniumoxid nachgegeben, nochmals 75 Minuten bei 95°C gerührt, 2.04 g Aktivkohle zugegeben und heiß filtriert. Das Filtrat wird über Ionenaustauscher wie in Beispiel 1c gereinigt und aus Wasser/Ethanol kristallisiert. Es werden 71.8 g ( 78% d. Th. ) des Gadolinium-Komplexes von 10-(1-Hydroxymethyl-2,3-dihydroxypropyl)-1,4,7-triscarboxymethyl-1,4,7,10-tetraazacydododecan erhalten.

| | | | | | | | | | |
|---|---|---|---|---|---|---|---|---|---|
| Elementaranalyse | Ber. | C | 35.75 | H | 5.17 | N | 9.27 | Gd | 26.00 |
| | Gef. | C | 35.67 | H | 5.19 | N | 9.27 | Gd | 25.99 |

### Beispiel 4

### Beispiel 4a

### 10-(2-Hydroxypropyl)-1,4,7,10-tetraazacyclododecan Tetrahydrochlorid

141.1 g (250 mmol) 1,4,7,10-Tetraazacyclododecantetrasulfat werden in 600 ml n-Butanol gelöst und unter Stickstoffatmosphäre mit 68 g Natriumhydroxid veresetzt. Das Gemisch wird erwärmt und azeotrop Wasser abdestilliert. Anschließend werden 20.0 g (350 mmol) Propylenoxid zugetropft, und nach beendeter Zugabe wird eine Stunde gekocht. Danach wird auf Raumtemperatur abgekühlt, mit 500 ml Wasser versetzt und 30 Minuten nachgerührt. Die werden getrennt und die Butanolphase mit 125 ml konzentrierter Salzsäure versetzt. Die Mischung wird auf 70°C erwärmt, eine Stunde nachgerührt und auf 100 ml im Vakuum eingeengt. Es werden 500 ml absolutes Methanol zugesetz. Nach nochmaligem Einengen werden 200 ml n-Butanol zugegeben und die Mischung in Eis/Wasser abgekühlt. Die ausfallenden Kristalle werden abgesaugt und zweimal mit Methyl-tert. butylether gewaschen. Nach Trocknen ( das Produkt verliert dabei HCI ) erhält man 70.5 g (75% d. Th.) weißkristallines Produkt.
Schmelzpunkt: 221°-224°C (Zersetzung)

| | | | | | | | | | |
|---|---|---|---|---|---|---|---|---|---|
| Elementaranalyse | Ber. | C | 35.12 | H | 8.04 | N | 14.89 | Cl | 37.69 |
| | Gef. | C | 37.23 | H | 8.36 | N | 15.68 | Cl | 32.61 |

### Beispiel 4b

### 10-(2-Hydroxypropyl)-1,4,7-triscarboxymethyl-1,4,7,10-tetraazacyclododecan

53 g (125 mmol) der in Beispiel 4a erhaltenen Verbindung werden analog zu Beispiel 1b umgesetzt. Es werden 56.5g (90% d. Th.) 10-(2-Hydroxypropyl)-1,4,7-triscarboxymethyl-1,4,7,10-tetraazacyclododecan erhalten.

| | | | | | | | |
|---|---|---|---|---|---|---|---|
| Elementaranalyse (auf Wasser korrigiert) | Ber. | C | 50.48 | H | 7.98 | N | 13.85 |
| | Gef. | C | 50.13 | H | 8.14 | N | 14.13 |

Wassergehalt: 10.1%

### Beispiel 5

### Beispiel 5a

### 10-(1-Hydroxymethyl-2,3-dihydroxypropyl)-1,4,7,10-tetraazacyclododecan Tetrahydrochlorid

Analog zu Beispiel la werden ausgehend von 192 g (500 mmol) 1,4,7,10-Tetraazacyclododecantetrahydrochlorid 183 g (71% d. Th.) 10-(1-Hydroxymethyl-2,3-dihydroxypropyl)-1,4,7,10-tetraazacyclododecan Tetrahydrochlorid erhalten.
Schmelzpunkt: 213°-215°C (Zersetzung)

### Beispiel 5b

### 10-(1-Hydroxymethyl-2,3-dihydroxypropyl)-1,4,7-triscarboxymethyl-1,4,7,10-tetraazacyclododecan

Analog zu Beispiel 1b werden ausgehend von 105.5 g (250 mmol) der aus Beispiel 5a erhaltenen Verbindung 110g (97% d. Th.) 10-(1-Hydroxymethyl -2,3-dihydroxypropyl)-1,4,7-triscarboxymethyl-1,4,7,10-tetraazacyclododecan erhalten.

| | | | | | | | |
|---|---|---|---|---|---|---|---|
| Elementaranalyse | Ber. | C | 47.98 | H | 7.61 | N | 12.43 |
| | Gef. | C | 47.38 | H | 7.62 | N | 12.37 |

### Beispiel 5c

### Gadolinium-Komplex von 10-(1-Hydroxymethyl-2,3-dihydroxypropyl)-1,4,7-triscarboxymethyl-1,4,7,10-tetraazacyclododecan

Analog zu Beispiel 1c werden aus 110g der aus Beispiel 5b erhaltenen Verbindung 98 g (66% d.Th.) des Gadolinium-Komplexes von10-(1-Hydroxymethyl-2,3-dihydroxypropyl)-1,4,7-triscarboxymethyl- 1,4,7,10-tetraazacyclododecan erhalten.
Schmelzpunkt: >300°C

### Beispiel 6

### 10-(6-Hydroxy-2,2-dimethyl-1,3-dioxepan-5-yl)-1,4,7,10-tetraazacyclododecan

121.5 g (250 mmol) 1,4,7,10-Tetraazacyclododecansulfat ( x 3,2 H₂SO₄) werden in 500 ml n-Butanol aufgeschlämmt und mit 48 g (1.2 mol) Natriumhydroxid versetzt. Das Gemisch wird langsam erwärmt und das entstehende Wasser azeotrop abdestilliert. Es wird eine Lösung von 4,4-Dimethyl-3,5,8-trioxabicyclo-[5,1,0]-octan in der entsprechenden bei der Destillation mit abdestillierten Menge n-Butanol zugegeben. Anschließend wird zwei Stunden am Rückfluß erhitzt. Das Gemisch wird abgekühlt und mit 300 ml Wasser versetzt. Die Butanolphase wird abgetrennt und eingeengt. Der Rückstand wird mit 100 ml Wasser versetzt und erneut eingeengt. Dieser Vorgang wird wiederholt. Es werden 135.1 g Rohprodukt erhalten, das in 600 ml Wasser gelöst und mit Ethylacetat gewaschen wird. Die vereinigten Ethylacetatphasen werden mit Wasser zurückggewaschen. Alle Wasserphasen werden vereinigt und eingeengt. Es werden 117.0 g 10-(6-Hydroxy-2,2-dimethyl-1,3-dioxepan-5-yl)-1,4,7,10-tetraazacyclododecan als Rohprodukt erhalten. Die weitere Umsetzung erfolgt wie in Beispiel 3 beschrieben.

### Beispiel 7

### Beispiel 7a

### 10-(6-Hydroxy-2,2-dimethyl-1,3-dioxepan-5-yl)-1,4,7,10-tetraazacyclododecan

10 kg 1,4,7,10-Tetraazacyclododecansulfat (x 3.2 H₂SO₄) werden zusammen mit 4 kg Natriumhydroxid in Plätzchen vorgelegt und 40 1 n-Butanol werden dazugepumpt. Unter Rühren und Stickstoffatmosphäre wird erhitzt und entstehendes Wasser azeotrop abdestilliert. Anschließend wird eine Lösung von 3.6 kg 4,4-Dimethyl-3,5,8-trioxabicyclo[5,1,0]-octan in 20 1 n-Butanol zugegeben und eine Stunde unter Rühren und Stickstoffatmosphäre am Rückfluß gekocht. Anschließend werden erneut 7 1 n-Butanol abdestilliert und 0.6 kg 4,4-Dimethyl-3,5,8-trioxabicyclo[5,1,0]-octan zugesetzt. Nach einer halben Stunde Erhitzen am Rückfluß unter Rühren und Stickstoffatmosphäre wird auf 40°C Innentemperatur gekühlt, mit 40 1 entionisiertem Wasser versetzt. Es werden die Phasen getrennt und die organische Phase mit 300 ml Eisessig und 10 1 entionisiertem Wasser versetzt. Nach 20 Minuten werden erneut die Phasen getrennt. Die wässrige Phase wird mit 100 ml 50%iger Natronlauge und 10 1 n-Butanol versetzt und 20 Minuten stehen gelassen. Die Phasen werden getrennt. Die organische Phase wird mit der bei der vorherigen Trennung erhaltenen zusammengefaßt und auf 10 1 eingeengt. Anschließend werden 50 1 entsalztes Wasser zugesetzt und das Gemisch auf ein Volumen von 45 l reduziert. Das Gemisch wird auf 20°C Innentemperatur gekühlt und währenddessen mit 1 l Essigsäure versetzt. Nach Zugabe von 10 1 Methylenchlorid oder Essigester wird 15 Minuten gerührt und dann 20 Minuten stehen gelassen. Es werden die Phasen getrennt. Die wässrige Phase wird noch zweimal mit je 10 1 Methylenchlorid extrahiert. Alle Methylenchloridphasen werden vereinigt, mit 10 1 entsalztem Wasser 15 Minuten gerührt und ca. 20 Minuten stehen gelassen. Die Phasen werden getrennt und die organische Phase auf ca. 5 1 eingeengt. Die wässrige Phase wird mit der bei der vorherigen Trennung erhaltenen vereinigt und im Vakuum bei 70°C Innentemperatur auf 40 1 eingeengt. Nach Abkühlen auf Raumtemperatur wird die erhaltene Lösung in die nächste Reaktion eingesetzt. Der Gehalt an Produkt wird von einer Probe über HPLC gegen externen Standard bestimmt. Der Gehalt beträgt 4.22 kg (65% d. Th.).

### Beispiel 7b

### 10-(1-Hydroxymethyl-2,3-dihydroxypropyl)-1,4,7-triscarboxymethyl-1,4,7,10-tetraazacyclododecan

10 kg 10-(1-Hydroxymethyl-2,3-dihydroxypropyl)-1,4,7,10-tetraazacyclododecan werden wie aus Beispiel 7a erhalten in wässriger Lösung vorgelegt und auf ein Gesamtvolumen von 80 1 mit entsalztem Wasser verdünnt. Es werden 13.6 kg Chloressigsäure zugegeben. Dann werden bei maximal 70°C Innentemperatur ca. 8 1 50%ige Natronlauge zugesetzt bis maximal ein pH-Wert von pH=10 erreicht ist. Der Ansatz wird sieben Stunden bei dieser Temperatur gerührt. Der pH-Wert wird ständig auf pH=9.5 gehalten. Danach werden nochmals 3.4 kg Chloressigsäure zugegeben. Es wird weitere drei Stunden bei 70°C gerührt und der pH-Wert konstant gehalten. Nach beendeter Umsetzung wird auf 50°C gekühlt und mit ca. 15 1 konzentrierter Salzsäure versetzt, wobei die Innentemperatur 60°C nicht übersteigen sollte, bis ein pH-Wert von pH=1 erreicht ist. Bei dieser Temperatur wird im Vakuum eingeengt. Der Rückstand wird mit 40 1 Methanol versetzt, erwärmt und unter Stickstoffatmosphäre 30 Minuten am Rückfluß erhitzt. Die auskristallisierten Salze werden abgetrennt und mit Methanol gewaschen. Die vereinigten Filtrate werden im Vakuum eingeengt, mit 80 l entionisiertem Wasser versetzt. Die Substanzlösung wird auf eine in üblicher Weise vorbereitete Ionenaustauschersäule ( Ionenaustauscher Amberlite® A 252 C, Volumen 165 l) gegeben und mit 10 1 entionisiertem Wasser nachgespült. In einer Rührblase werden 600 1 entionisiertes Wasser mit 150 l 25%iger Ammoniaklösung versetzt. Der so verdünnte Ammoniak wird auf die Ionenaustauschersäulen gegeben und das Eluat in 50 1 Fraktionen gesammelt. Die produkthaltigen Fraktionen werden vereinigt und zunächst auf 50 1 und dann bei maximal 50°C Manteltemperatur im Vakuum eingeengt. Der Rückstand wird in 30 1 entionisiertem Wasser aufgenommen. Die so erhaltene Lösung wird direkt in die nächste Stufe eingesetzt. Die im Laufe der Reaktion abdestillierten Lösungsmittel sind für weitere Reaktionen wieder verwendbar.

### Beispiel 7c

### Gadolinium-Komplex von 10-(1-Hydroxymethyl-2,3-dihydroxypropyl)-1,4,7-triscarboxymethyl-1,4,7,10-tetraazacyclododecan

Die aus aus Beispiel 7b erhaltene Lösung wird vorgelegt und gegebenenfalls auf ein Volumen von 24 1 verdünnt. Dann werden 2.3 kg Gadoliniumoxid zugegeben. Das Gemisch wird unter Rühren auf 90°C erwärmt und der pH-Wert gegebenenfalls mit ca. 0.6 1 Essigsäure auf pH=6-7 eingestellt. Wenn eine Lösung entstanden ist (unlösliche Feststoffe werden gegebenenfalls abfiltriert) wird eine Stunde bei 90°C gerührt. Der pH-Wert wird währenddessen gehalten. Nach beendeter Umsetzung wird auf 20°C gekühlt und 0.3 kg Aktivkohle zugesetzt. Es wird eine Stunde bei 20°C gerührt und die Aktivkohle abfiltriert. Die Aktivkohle wird substanzfrei gewaschen und die Lösung mehrfach filtriert. Anschließend wird die Lösung in dem Fachmann bekannter Weise über Ionenaustauscher gereinigt bis im Eluat keine Leitfähigkeit mehr festgestellt werden kann und aus Ethanol/Wasser kristallisiert und getrocknet. Es werden 5.24 kg ( das entspricht einer Gesamtausbeute über alle Stufen von 42% d. Th.) des Gadolinium-Komplexes von 10-(1-Hydroxymethyl-2,3-dihydroxypropyl)-1,4,7-triscarboxymethyl-1,4,7,10-tetraazacyclododecan erhalten.

| | | | | | | | | | |
|---|---|---|---|---|---|---|---|---|---|
| Elementaranalyse | Ber. | C | 35.75 | H | 5.17 | N | 9.27 | Gd | 26.00 |
| | Gef. | C | 35.69 | H | 5.21 | N | 9.25 | Gd | 25.98 |

### Beispiel 8

### Beispiel 8a:

### 10-(2-Hydroxy-(2,2-dimethyl-1,3-dioxolan-4-yl)ethyl)-1,4,7,10-tetraazacyclododecan

Analog zu Beispiel 3a werden 124 g (250 mmol) 1,4,7,10-Tetraazacyclododecansulfat ( x 3,3 H₂SO₄) mit insgesamt 50.45g (350 mmol) 2,2-Dimethyl-4-(2',3'-epoxy)-propoxy-methyl-1,3-dioxolan umgesetzt. Man erhält 59.3g (75% d. Th.) 10-(2-Hydroxy-(2,2-dimethyl-1,3-dioxolan-4-yl)ethyl)-1,4,7,10-tetraazacyclododecan das sofort weiter umgesetzt wird.

### Beispiel 8b

### 10-(2,3,4-Trihydroxybutyl)-1,4,7-triscarboxymethyl-1,4,7,10-tetraazacyclododecan

59.3g der aus Beispiel 8a erhaltenen Verbindung werden analog Beispiel 3b umgesetzt. Es werden 63.7g (75% d. Th.) 10-(2,3,4-Trihydroxybutyl)-1,4,7-triscarboxymethyl-1,4,7,10-tetraazacyclododecan erhalten.

| | | | | | | | |
|---|---|---|---|---|---|---|---|
| Elementaranalyse | Ber. | C | 47.98 | H | 7.61 | N | 12.43 |
| | Gef. | C | 47.21 | H | 7.64 | N | 12.92 |

### Beispiel 8c

### Gadolinium-Komplex von 10-(2,3,4-Trihydroxybutyl)-1,4,7-triscarboxymethyl-1,4,7,10-tetraazacyclododecan

68.0g der aus Beispiel 8b erhaltenen Verbindung werden analog Beispiel 3c umgesetzt. Es werden 89.3g (97% d. Th.) des Gadolinium-Komplexes von 10-(2,3,4-Trihydroxybutyl)-1,4,7-triscarboxymethyl-1,4,7,10-tetraazacyclododecan erhalten.

| | | | | | | | | | |
|---|---|---|---|---|---|---|---|---|---|
| Elementaranalyse | Ber. | C | 35.75 | H | 5.17 | N | 9.27 | Gd | 26.00 |
| | Gef. | C | 35.64 | H | 5.23 | N | 9.23 | Gd | 26.02 |

## Patentansprüche

1. Verfahren zur Herstellung von N-β-Hydroxyalkyl-tri-N-carboxyalkyl-1,4,7,10-tetraazacyclododecan- und N-β-Hydroxyalkyl-tri-N-carboxyalkyl-1,4,8,11-tetraazacyclotetradecan-Derivaten der allgemeinen Formel I 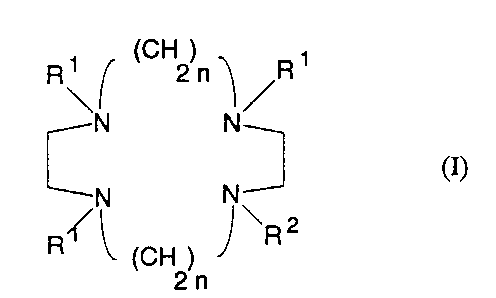 worin
R¹ eine gegebenenfalls durch R³ substituierte -(CH₂)₁₋₆-COOY-Gruppe mit
R³ in der Bedeutung eines Wasserstoffatoms, einer C₁-C₆ Alkylgruppe, einer Benzyl-, einer Benzyloxyalkyl- oder einer Phenylgruppe, und
Y in der Bedeutung eines Wasserstoffatoms und/oder eines Metallionenäquivalentes eines Elements der Ordnungszahlen 21-29, 31, 32, 37-39, 42-44, 49 oder 57-83
R² eine 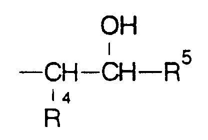 Gruppe
n die Ziffern 2 oder 3
R⁴ und R⁵ unabhängig voneinander jeweils für ein Wasserstoffatom, einen gegebenenfalls durch 1 bis 10 Sauerstoffatom(e), eine Phenylen-, Phenylenoxyoder Phenylendioxygruppe unterbrochenen C₁-C₂₀ Alkylrest, der gegebenenfalls durch 1 bis 3 C₁-C₆-AJkyl, 1 bis 3 Trifluormethyl-, 1 bis 7 Hydroxy-, 1 bis 3 C₁-C₇-AJkoxy- oder -(C₆-C₁₀)Ar-(C₁-C₆)-alkoxy-, 1 bis 2 CO₂R⁶-Reste,
wobei
R⁶ für ein Wasserstoffatom, eine C₁-C₆-Alkylgruppe, eine C₆-C₁₀-Aryl- oder eine C₆-C₁₀-Ar(C₁-C₄)-alkylgruppe steht,
und/oder 1 bis 2 gegebenenfalls durch 1 bis 2 Chlor-, Brom-, Nitro- oder C₁-C₆-Alkoxyreste substituierte Phenoxy- oder Phenylgruppen substituiert ist,
bedeuten, wobei die gegebenenfalls vorhandenen Hydroxyreste oder Carboxygruppen gegebenenfalls in geschützter Form vorliegen, mittels Epoxideinsatz, dadurch gekennzeichnet, daß man 1,4,7,10-Tetraazacyclododecan oder 1,4,8,11-Tetraazacyclotetradecan, gegebenenfalls in Form ihrer Salze, in Gegenwart einer Base mit einem Epoxid der Formel II, 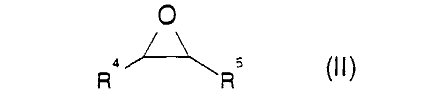 worin
R⁴ und R⁵ die oben angegebenen Bedeutungen haben, wobei gegebenenfalls vorhandene Hydroxy- oder Carboxygruppen gegebenenfalls geschützt sind,
in einem polaren Lösungsmittel oder ohne Lösungsmittel bei Temperaturen von 0°-220°C innerhalb von 0.5 - 48 Stunden umsetzt, die Verunreinigungen abtrennt, gewünschtenfalls, wenn notwendig nach Zugabe von Säuren, isoliert, und gegebenenfalls in Gegenwart einer Base, mit einer Verbindung der Formel III 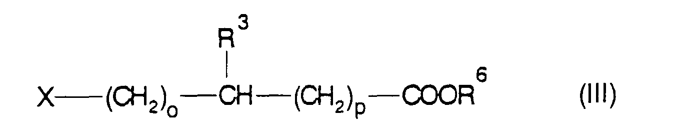 worin
R³ und R⁶ die oben angegebene Bedeutung haben,
X eine Fluchtgruppe, und
o,p unabhängig voneinander die Ziffern 0 bis 5, wobei die Summe aus o+p<6 sein muß, bedeuten,
gegebenenfalls nach Schutz von Hydroxy- oder Carboxygruppen in R², in einem polaren Lösungsmittel bei -10°C bis 170°C innerhalb von 1 - 100 Stunden umsetzt, gegebenenfalls Schutzgruppen abspaltet und das so erhaltene Produkt der Formel I, in dem Y Wasserstoff bedeutet, gewünschtenfalls in an sich bekannter Weise mit mindestens einem Metalloxid oder Metallsalz eines Elements der Ordnungszahlen 21-29, 31, 32, 37-39, 42-44, 49 oder 57-83 umsetzt, falls gewünscht, noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt und den so erhaltenen Komplex isoliert.

2. Verfahren gemäß Anspruch 1, dadurch gekennzeichnet, daß Salze anorganischer oder organischer Säuren des 1,4,7,10-Tetraazacyclododecans oder des 1,4,8,11-Tetraazacyclotetradecans als Ausgangsmaterial verwendet werden.

3. Verfahren gemäß Anspruch 2 dadurch gekennzeichnet, daß Hydrochloride oder Sulfate des 1,4,7,10-Tetraazacyclododecans oder des 1,4,8,11-Tetraazacyclotetradecans eingesetzt werden.

4. Verfahren gemäß Anspruch 2 dadurch gekennzeichnet, daß Hydrochloride oder Sulfate des 1,4,7,10-Tetraazacyclododecans eingesetzt werden.

5. Verfahren gemäß Anspruch 1 dadurch gekennzeichnet, daß die Base für die Umsetzung mit dem Epoxid Lithium-, Natrium- oder Kaliumhydroxid ist.

6. Verfahren gemäß Anspruch 1 dadurch gekennzeichnet, daß R⁴ und R⁵ unabhängig voneinander ein Wasserstoffatom oder eine gegebenenfalls durch 1 - 4 Hydroxygruppen substituierte C₁ - C₄ Alkylgruppe bedeuten.

7. Verfahren gemäß Anspruch 1 dadurch gekennzeichnet, daß R⁴ ein Wasserstoffatom, eine Methylgruppe oder eine Hydroxymethylgruppe und R⁵ eine Methylgruppe, eine Hydroxymethylgruppe oder eine 1,2-Dihydroxyethylgruppe bedeuten.

8. Verfahren gemäß Anspruch 1 dadurch gekennzeichnet, daß das Verhältnis von Epoxid zum Tetraazamakrocyclus 1:1 bis 2:1 beträgt.

9. Verfahren gemäß Anspruch 1 dadurch gekennzeichnet, daß das Lösungsmittel für die Umsetzung mit dem Epoxid polar oder polar und protisch ist.

10. Verfahren gemäß Anspruch 9 dadurch gekennzeichnet, daß das Lösungsmittel n-Butanol ist.

11. Verfahren gemäß Anspruch 1 dadurch gekennzeichnet, daß die Umsetzung mit dem Epoxid bei Temperaturen zwischen Raumtemperatur und 200°C erfolgt.

12. Verfahren gemäß Anspruch 1 dadurch gekennzeichnet, daß die Umsetzung mit dem Epoxid bei Temperaturen von 50°C bis 150°C erfolgt.

13. Verfahren gemäß Anspruch 1 dadurch gekennzeichnet, daß eine Verbindung der Formel III eingesetzt wird, worin R³ für ein Wasserstoffatom steht und o=p=0 bedeutet.

14. Verfahren gemäß Anspruch 1 dadurch gekennzeichnet, daß die Reaktionen in einem Eintopfverfahren durchgeführt werden.

## Claims

1. Process for preparing N-β-hydroxyalkyl-tri-N-carboxyalkyl-1,4,7,10-tetraazacyclododecane and N-β-hydroxyalkyl-tri-N-carboxyalkyl-1,4,8,11-tetraazacyclotetradecane derivatives of the general formula I 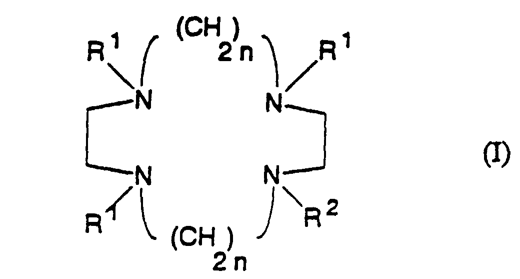 in which
R¹ denotes an optionally R³-substituted -(CH₂)₁₋₆-COOY-group
where
R³ denotes a hydrogen atom, a C₁-C₆-alkyl group, a benzyl, benzyloxyalkyl or a phenyl group, and
Y denotes a hydrogen atom and/or a metal ion equivalent of an element of the atomic numbers 21-29, 31, 32, 37-39, 42-44, 49 or 57-83,
R² denotes a 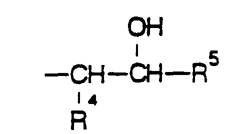 group
n denotes the numbers 2 or 3
R⁴ and R⁵ independently of one another each denote a hydrogen atom or a C₁-C₂₀-alkyl radical which is optionally interrupted by 1 to 10 oxygen atom(s), a phenylene, phenyleneoxy or phenylenedioxy group and which is optionally substituted by 1 to 3 C₁-C₆-alkyl, 1 to 3 trifluoromethyl, 1 to 7 hydroxyl, 1 to 3 C₁-C₇-alkoxy or C₆-C₁₀-Ar-C₁-C₆-alkoxy, 1 to 2 CO₂R⁶ radicals,
where
R⁶ denotes a hydrogen atom, a C₁-C₆-alkyl group, a C₆-C₁₀-aryl or a C₆-C₁₀-Ar-C₁-C₄-alkyl group,
and/or 1 to 2 phenoxy or phenyl groups which are optionally substituted by 1 to 2 chlorine, bromine, nitro or C₁-C₆-alkoxy radicals,
where the hydroxyl radicals or carboxyl groups which are optionally present may optionally be present in protected form, by use of epoxide,
characterized in that 1,4,7,10-tetraazacyclododecane or 1,4,8,11-tetraazacyclotetradecane, if appropriate in the form of their salts, are reacted in the presence of a base with an epoxide of the formula II 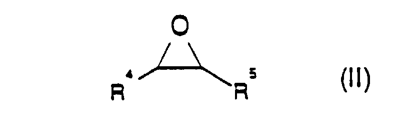 in which
R⁴ and R⁵ are as defined above, hydroxyl or carboxyl groups which are optionally present being optionally protected,
in a polar solvent or in the absence of a solvent at temperatures of 0°-220°C over a period of 0.5-48 hours, the impurities are removed, the product is, if desired, if necessary after addition of acids, isolated and, optionally in the presence of a base, reacted with a compound of the formula III 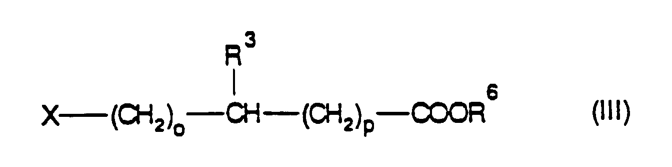 in which
R³ and R⁶ are as defined above,
X denotes a leaving group, and
o and p independently of one another denote the numbers 0 to 5, where the sum of o + p has to be <6,
if appropriate after protecting the hydroxyl or carboxyl groups in R², in a polar solvent at from -10°C to 170°C over a period of 1-100 hours, protective groups are, if appropriate, removed and the resulting product of the formula I in which Y denotes hydrogen is, if desired, reacted in a manner known per se with at least one metal oxide or metal salt of an element of the atomic numbers 21-29, 31, 32, 37-39, 42-44, 49 or 57-83, any acidic hydrogen atoms which may still be present are, if desired, substituted by cations of inorganic and/or organic bases, amino acids or amino acid amides and/or some or all of the corresponding acid groups are converted into esters or amides and the resulting complex is isolated.

2. Process according to Claim 1, characterized in that salts of inorganic or inorganic acids of 1,4,7,10-tetraazacyclododecane or 1,4,8,11-tetraazacyclotetradecane are used as starting material.

3. Process according to Claim 2, characterized in that hydrochlorides or sulphates of 1,4,7,10-tetraazacyclododecane or 1,4,8,11-tetraazacyclotetradecane are used.

4. Process according to Claim 2, characterized in that hydrochlorides or sulphates of 1,4,7,10-tetraazacyclododecane are used.

5. Process according to Claim 1, characterized in that the base used for the reaction with the epoxide is lithium hydroxide, sodium hydroxide or potassium hydroxide.

6. Process according to Claim 1, characterized in that R⁴ and R⁵ independently of one another denote a hydrogen atom or a C₁-C₄-alkyl group which is optionally substituted by 1-4 hydroxyl groups.

7. Process according to Claim 1, characterized in that R⁴ denotes a hydrogen atom, a methyl group or a hydroxymethyl group and R⁵ denotes a methyl group, a hydroxymethyl group or a 1,2-dihydroxyethyl group.

8. Process according to Claim 1, characterized in that the ratio of epoxide to tetraazamacrocycle is 1:1 to 2:1.

9. Process according to Claim 1, characterized in that the solvent used for the reaction with the epoxide is polar or polar and protic.

10. Process according to Claim 9, characterized in that the solvent used is n-butanol.

11. Process according to Claim 1, characterized in that the reaction with the epoxide is carried out at temperatures between room temperature and 200°C.

12. Process according to Claim 1, characterized in that the reaction with the epoxide is carried out at temperatures of from 50°C to 150°C.

13. Process according to Claim 1, characterized in that a compound of the formula III is used in which R³ denotes a hydrogen atom and o=p=0.

14. Process according to Claim 1, characterized in that the reactions are carried out in a one-pot process.

## Revendications

1. Procédé de préparation de dérivés de N-β-hydroxyalkyl-tri-N-carboxyalkyl-1,4,7,10-tétraazacyclododécane et de N-β-hydroxyalkyl-tri-N-carboxyalkyl-1,4,8,11-tétra-azacyclotétradécane de formule générale I 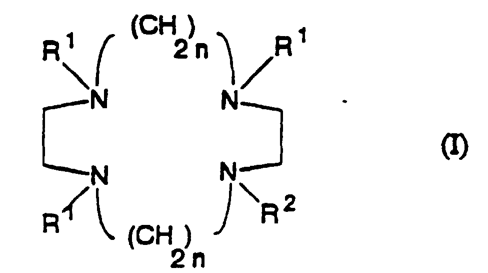 dans laquelle
R¹ représente un groupe -(CH₂)₁₋₆-COOY éventuellement substitué par R³ avec
R³ représentant un atome d'hydrogène, un groupe alkyle en C₁-C₆, un groupe benzyle, benzyloxy alkyle ou phényle, et
Y représentant un atome d'hydrogène et/ou un équivalent d'ion métallique d'un élément de numéros atomiques 21-29, 31, 32, 37-39, 42-44, 49 ou 57-83
R² représente un groupe 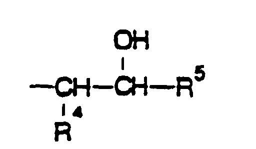
n représente les chiffres 2 ou 3
R⁴ et R⁵ représentent chacun, indépendamment l'un de l'autre, un atome d'hydrogène, un radical alkyle en C₁-C₂₀ éventuellement interrompu par 1 à 10 atome(s) d'oxygène, un groupe phénylène, phénylène-oxy ou phénylène-dioxy, et qui est éventuellement substitué par 1 à 3 radicaux alkyle en C₁-C₆, 1 à 3 radicaux trifluorométhyle, 1 à 7 radicaux hydroxy, 1 à 3 radicaux alcoxy en C₁-C₇ ou -(C₆-C₁₀)Ar-(C₁-C₆)-alcoxy, 1 à 2 radicaux CO₂R⁶,
où
R⁶ désigne un atome d'hydrogène, un groupe alkyle en C₁-C₆, un groupe aryle en C₆-C₁₀ ou un groupe C₆-C₁₀-Ar(C₁-C₄)-alkyle,
et/ou 1 à 2 groupes phénoxy ou phényle éventuellement substitués par 1 à 2 radicaux chloro, bromo, nitro ou alcoxy en C₁-C₆,
les radicaux hydroxy ou groupes carboxy éventuellement présents se trouvant éventuellement sous forme protégée, en utilisant de l'époxyde
caractérisé en ce que l'on fait réagir du 1,4,7,10-tétra-azacyclododécane ou du 1,4,8,11-tétraazacyclotétradécane, éventuellement sous forme de leurs sels, en présence d'une base, avec un époxyde de formule II, 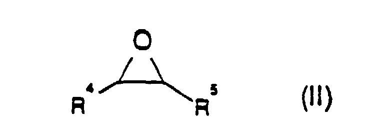 dans laquelle
R⁴ et R⁵ ont les significations mentionnées ci-dessus, les groupes carboxy ou hydroxy éventuellement présents étant éventuellement protégés,
dans un solvant polaire ou en l'absence de solvants, à des températures de 0°-220°C, sur une période de 0,5-48 heures, on sépare les impuretés, si on le souhaite, on procède à l'isolation, le cas échéant après addition d'acides, et on met éventuellement à réagir, en présence d'une base, avec un composé de formule III 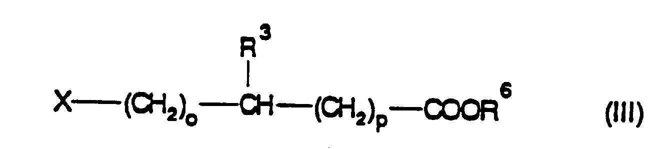 dans laquelle
R³ et R⁶ ont les significations mentionnées ci-dessus
X représente un groupe partant, et
o, p représentent, indépendamment l'un de l'autre, les chiffres 0 à 5, la somme de 0 + p devant être < 6,
éventuellement après protection des groupes hydroxy ou carboxy de R², dans un solvant polaire, entre -10°C et 170°C, sur une période de 1-100 heures, on élimine éventuellement les groupes protecteurs et on met éventuellement à réagir le produit ainsi obtenu de formule I, dans laquelle Y représente un hydrogène, si on le souhaite d'une manière connue en soi, avec au moins un oxyde métallique ou un sel métallique d'un élément de numéros atomiques 21-29, 31, 32, 37-39, 42-44, 49 ou 57-83, si on le souhaite, on remplace les atomes d'hydrogène acides encore présents par des cations de bases inorganiques et/ou organiques, d'amino-acides ou d'amides d'amino-acides ou on transforme les groupes acides correspondants partiellement ou totalement en esters ou en amides, et on isole le complexe ainsi obtenu.

2. Procédé selon la revendication 1, caractérisé en ce que l'on utilise des sels d'acides inorganiques ou organiques du 1,4,7,10-tétra-azacyclododécane ou du 1,4,8,11-tétra-azacyclotétradécane en tant que composé de départ.

3. Procédé selon la revendication 2, caractérisé en ce que l'on utilise des chlorhydrates ou des sulfates du 1,4,7,10-tétra-azacyclododécane ou du 1,4,8,11-tétra-azacyclotétradécane.

4. Procédé selon la revendication 2, caractérisé en ce que l'on utilise des chlorhydrates ou des sulfates du 1,4,7,10-tétra-azacyclododécane.

5. Procédé selon la revendication 1, caractérisé en ce que la base pour le réaction avec l'époxyde est l'hydroxyde de lithium, de sodium ou de potassium.

6. Procédé selon la revendication 1, caractérisé en ce que R⁴ et R⁵ représentent, indépendamment l'un de l'autre, un atome d'hydrogène ou un groupe alkyle en C₁-C₄ éventuellement substitué par 1-4 groupes hydroxy.

7. Procédé selon la revendication 1, caractérisé en ce que R⁴ représente un atome d'hydrogène, un groupe méthyle ou un groupe hydroxyméthyle et R⁵ représente un groupe méthyle, un groupe hydroxyméthyle ou un groupe 1,2-dihydroxyéthyle.

8. Procédé selon la revendication 1, caractérisé en ce que le rapport entre l'époxyde et le tétraazamacrocyle est de 1:1 à 2:1.

9. Procédé selon la revendication 1, caractérisé en ce que le solvant pour la réaction avec l'époxyde est polaire ou polaire et protique.

10. Procédé selon la revendication 9, caractérisé en ce que le solvant est le n-butanol.

11. Procédé selon la revendication 1, caractérisé en ce que la réaction avec l'époxyde a lieu à des températures comprises entre la température ambiante et 200°C.

12. Procédé selon la revendication 1, caractérisé en ce que la réaction avec l'époxyde a lieu à des températures allant de 50°C à 150°C.

13. Procédé selon la revendication 1, caractérisé en ce que l'on utilise un composé de formule III, dans laquelle R³ désigne un atome d'hydrogène et o=p=0.

14. Procédé selon la revendication 1, caractérisé en ce que les réactions sont réalisées en un procédé à une seule étape.
